# EUROPÄISCHE PATENTSCHRIFT

(11) **EP 1 227 181 B1**
(45) Veröffentlichungstag und Bekanntmachung des Hinweises auf die Patenterteilung: **29.11.2017**
(21) Anmeldenummer: 01130149.6
(22) Anmeldetag: 19.12.2001
(51) Int. Cl.: B29C 43/22, B29C 59/04, D04H 1/559, D04H 3/02, D04H 1/541, D04H 1/54, B29K 105/08, A61F 13/511, A61F 13/513

(54) **Vliesstoff mit Struktur**
Three-dimensional nonwoven
Non-tissé tridimensionnel

(30) Priorität: 27.01.2001 DE 10103627
(43) Veröffentlichungstag der Anmeldung: 31.07.2002
(73) Patentinhaber: Sandler AG, 95126 Schwarzenbach/Saale (DE)
(72) Erfinder:

(56) Entgegenhaltungen:
- EP-A- 0 893 529
- WO-A-95/07673
- WO-A-97/02133
- CH-A- 498 234
- DE-A- 19 927 785
- DE-U- 29 723 320
- US-A- 3 993 820
- US-A- 5 128 193
- US-A- 5 531 727

## Beschreibung

Die Strukturgebung an Vliesstoffen, speziell an kalandrierten Vliesstoffen, sogenannten Thermobondings, ist ein allgemein übliches Verfahren.

Neben dem grundlegenden Verfahren zur Verfestigung von Faserfloren hin zum Vlies mittels eines Kalanders, wie in Albrecht, "Vliesstoffe", erschienen bei Wiley-VCH, 2000, Seite 363 bis 368, beschrieben, gab es immer wieder Anstrengungen, funktionale, gegen mechanische Beanspruchungen beständige Strukturen in einen Thermobonding-Vliesstoff einzubringen.

So ist beispielsweise aus der EP 0 882 828 A1 ein Verfahren zur Herstellung eines Schlaufenmaterials bekannt, nach welchem ein Verbund einer Faser- oder Filamentschicht mit einem Trägermaterial mittels eines Kalanderverfahrens unter Benutzung eines definierten Prägemusters hergestellt wird. Die Verwendung eines Trägermaterials ist für die mechanische Stabilität des erhaltenen Verbundes unabdingbar, da ansonsten das Material im Einsatz zu schnell zerstört wird. Diese Arbeitsweise bedingt aber einen mehrstufigen Arbeitsablauf und ist somit wirtschaftlich uninteressant.

In der EP 0 736 114 B1 wird beschrieben, wie ein Thermobonding-Vliesstoff einen textilähnlichen Griff aufgrund eingebrachter, definierter Prägemuster erhält. Dieses Verfahren beeinflusst allerdings nur den Griff eines Vliesstoffes und bringt keine zusätzliche Funktion ein.

Die US 5 858 515 und US 5 962 112 zeigen Wege auf, wie man einen Thermobonding-Vliesstoff als Reinigungsmaterial einsetzen kann. Die eingebrachte Punktstruktur hat die Aufgabe eine Scheuerwirkung zu erzielen. Die beschriebenen Prägestrukturen sind aber bezüglich ihrer Speicherwirkung für Schmutzpartikel nur unzureichend und auch mechanisch instabil, das heißt, sie sind zu leicht zusammendrückbar.

Die EP 0 328 785 B1 zeigt eine Arbeitsweise, mit welcher es möglich ist, einen dreidimensional geformten, thermisch kalanderverfestigten Vliesstoff herzustellen. Hier wird mittels zweier, sich zueinander im Eingriff befindlicher, Kalanderwalzen ein Faserflor an den Berührungsstellen der Kalanderwalzen verfestigt. Das Fasermaterial, welches sich in den Zwischenbereichen der Kalanderwalzen befindet, wird nicht weiter beeinträchtigt. Ein nach diesem Verfahren hergestelltes Vlies zeigt in der Seitenansicht eine "Trapezblech"-ähnliche Struktur und kann beispielsweise als Saugvlies im Hygienebereich eingesetzt werden.

Eine ähnliche Verfahrensweise wird auch in der EP 0 810 078 A1 beschrieben, allerdings hier ohne eine thermische Fixierung der erhaltenen Struktur.

Nachteilig ist bei beiden aber die mangelnde Beständigkeit gegenüber mechanischen Belastungen, die dreidimensionale Form fällt bei Druckbelastungen sofort zusammen und erholt sich nicht mehr.

Gerade im Bereich der Hygieneartikel gab es zahlreiche Anstrengungen, mittels strukturierter Vliesstoffe, Funktionalitäten im Bereich der Aufnahme und Verteilung von Fluiden, hauptsächlich Körperexudaten, in einen Thermobonding-Vliesstoff einzubringen.

Die Funktion einer sogenannten Aufnahme- und Verteilungslage in einem Hygiene-Artikel ist, die abgegebene Fluide möglichst schnell von der dem Träger zugewandten flüssigkeitsdurchlässigen Abdeckschicht aufzunehmen, über eine bestimmte Fläche kontrolliert zu verteilen und an den nachgeordneten Saugkörper zur Speicherung abzugeben.

Eine mehrmalige Flüssigkeitsbeaufschlagung darf die Wirksamkeit der Aufnahme- und Verteilungslage nicht wesentlich beeinträchtigen, dazu wurde eine Vielzahl von als Aufnahme- und Verteilungslage verwendeten Vliesstoffen mit unterschiedlichen Materialzusammensetzungen und Konstruktionen beschrieben

Die US 5,968,855 offenbart eine Flüssigkeits-Transportlage für Hygiene-Artikel, bestehend aus einem kardierten und thermobondierten Vliesstoff, der sich aus einer Mischung aus niedrig- und hochschmelzenden polymeren Fasern mit unterschiedlichen Feinheiten zusammensetzt.

Diese Flüssigkeits-Transportslage besitzt aufgrund ihrer Konsistenz zwar eine ausreichende Aufnahmegeschwindigkeit der abgegebenen Körperflüssigkeit, verfügt jedoch nicht über die Fähigkeit, die Flüssigkeit auf die gewünschte Fläche gezielt und kontrolliert zu verteilen.

Die Verteilung von Fluiden erfolgt lediglich in sich konzentrisch ausbreitender Richtung und läßt sich mit der beschriebenen Struktur nicht kontrolliert steuern.

Die EP 0 842 650 A1 offenbart ein poröses Material zur Flüssigkeitsaufnahme und - verteilung in Hygiene-Artikeln.

Dieses poröse Material besteht aus einem kardierten Vliesstoff, der mit Hilfe von chemischen Bindemitteln verfestigt wird, wofür entweder ein Styrene-Butadiene-Rubber oder ein Ethylene-Vinyl-Acetate oder ein Ethylene-Vinyl-Alcohol oder ein Acrylic-Based-Resin verwendet wird.

Da hier auf wässriger Basis gearbeitet wird, ist der Energieaufwand für die Trocknung des fertigen Produktes enorm. Die EP bietet daher unter den ständig steigenden umwelttechnischen Anforderungen keinesfalls eine optimale Lösung.

In der EP 0 767 649 B1 wird ein absorbierendes Hygieneprodukt offenbart, welches eine flüssigkeitsdurchlässige Deckschicht aus einem Faservliesmaterial enthält. Diese Deckschicht ist in Längsrichtung des Produktes faltenartig angeordnet und an den Bodenfeldern der Falten mit dem absorbierenden Körper verbunden.

Neben der Tatsache, dass hier zweilagig mit einem zweiten Arbeitsgang gearbeitet werden muss, ist der gravierendste Nachteil dieser Faltenstruktur darin zu sehen, dass nach erfolgter Fluidbeaufschlagung ein beträchtlicher Verlust an mechanischer Stabilität erfolgt.

Unter Druckeinwirkung werden die Falten niedergedrückt und bleiben flach an der Oberfläche des Saugkörpers liegen. Da sie sich nach einer entsprechenden Druckbelastung nicht erneut aufrichten, erhöht sich die Ansaugzeit für die Flüssigkeitsaufnahme erheblich und die Verteilungsfunktion geht weitgehend verloren.

Dies kann im Extremfall zum Leakage und damit unerwünschten Rücktransport der Körperflüssigkeit auf die Hautoberfläche führen.

Der Tragekomfort des Hygiene-Artikels ist dann nicht mehr gewährleistet und Hautirritationen können hervorgerufen werden.

### Aufgabe der Erfindung

Aufgabe der Erfindung war es daher, einen thermisch kalanderverfestigten Vliesstoff zu schaffen, der eine mechanisch stabile, funktionale Struktur aufweist und die vorgenannten Nachteile des Standes der Technik vermeidet.

Die Aufgabe wird gemäß den Merkmalen des Anspruches 1 gelöst.

### Beschreibung der Erfindung

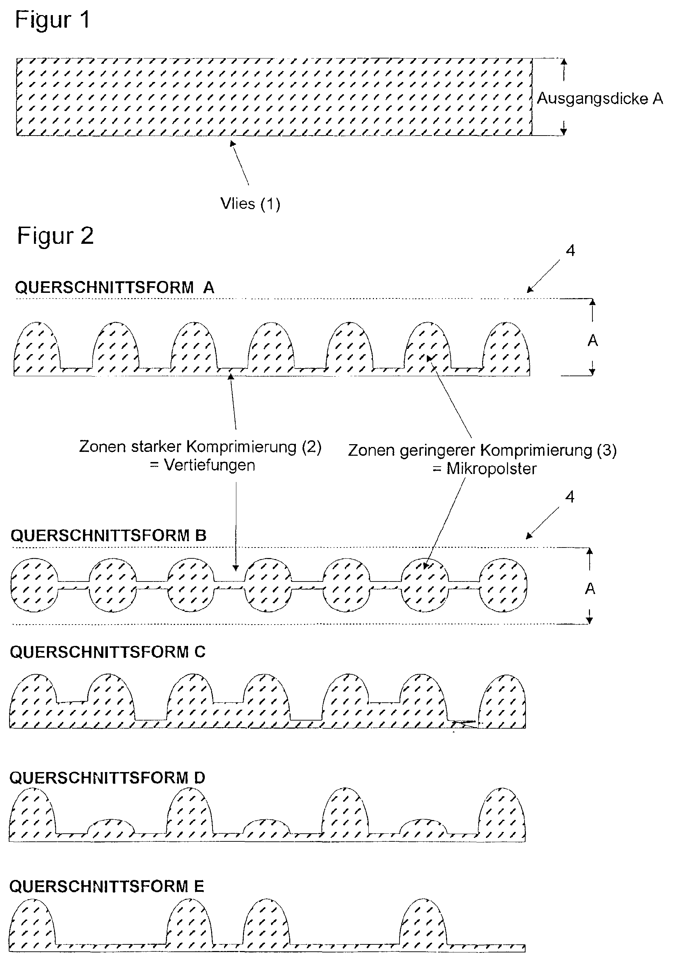

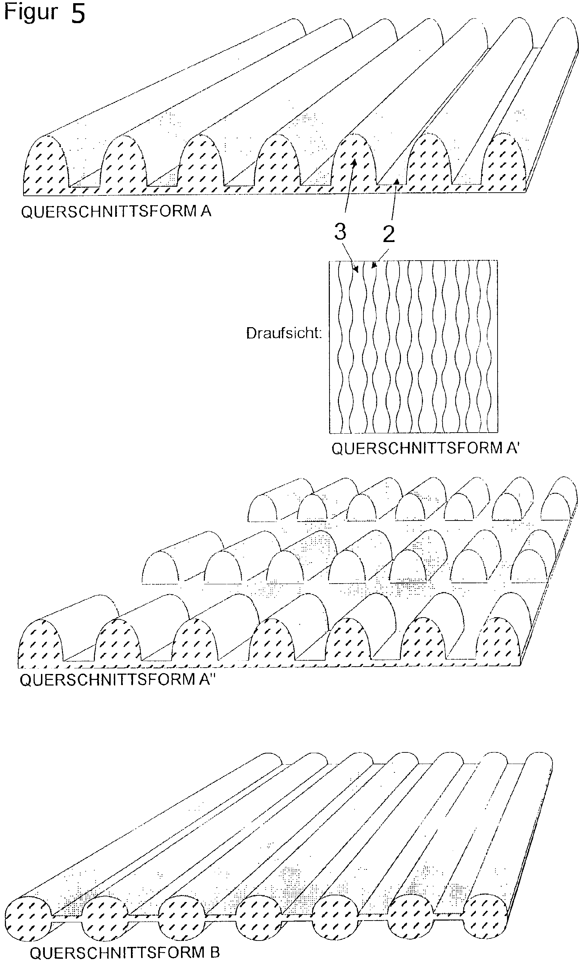

In ein Vlies (1) werden mittels eines Kalandriervorganges thermisch die für den Einsatzzweck notwendigen, mechanisch stabilen Strukturen eingeprägt. Mögliche Formen sind in der Figur 2 als Querschnitte und der Figur 5 räumlich dargestellt.

Ein mögliches Verfahren zur Herstellung des erfindungsgemäßen Vliesstoffes mit Strukur (4) ist nachstehend beschrieben.

Für den Vliesstoff (4) der Erfindung wird beispielsweise als Ausgangspunkt eine Fasermischung aus einer bikomponenten Faser mit einer Feinheit von 5,3 dtex, welche einen Polyethylenmantel und einen Polyethylenterephthalatkern aufweist, und einer Polyethylenterephthalat -Homopolymer-Faser mit einer Feinheit von 7,0 dtex hergestellt. Das Mischungsverhältnis liegt dabei bei 30% für die Bikomponentenfaser und bei 70% für die Homopolymerfaser.

Prinzipiell kann aber für die Fasermischung jede andere thermoplastische Faser, beispielsweise auf Basis von Polyolefinen oder Polyamiden, ob homopolymer oder multikomponent mit beliebigen Mischungsverhältnissen eingesetzt werden.

Diese Fasermischung wird mittels eines gängigen Krempelverfahrens, wie beispielsweise in Albrecht, "Vliesstoffe", Seite 145 ff beschrieben, zu einem Faserflor (5) mit einem mittleren Florgewicht von zum Beispiel 64 g/qm verarbeitet.

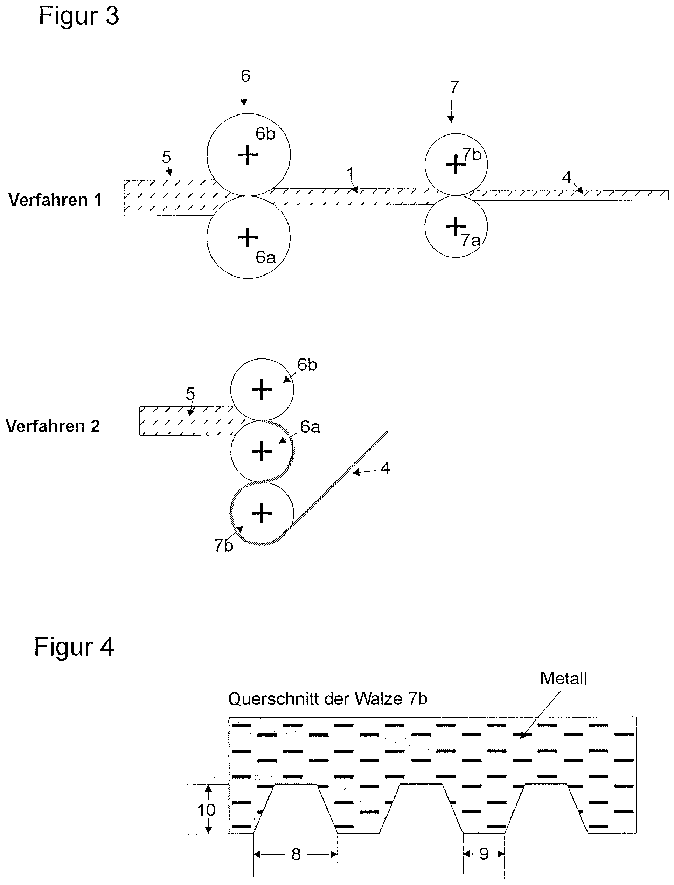

Der so erhaltene Faserflor wird dann einer Maschinenkombination wie beispielsweise in Figur 3 als Verfahren 1 gezeigt, zugeführt, welche im einfachsten Fall aus einem Prägekalander (6) zur Verfestigung des Faserflores zum Vlies (1) und einem Strukturkalander (7) zur Ausbildung der funktionalen Struktur des Vliesstoffes (4) zugeführt. Auch eine Zusammenführung beider Kalander in einen Aufbau ist, wie in Verfahren 2 von Figur 3 gezeigt, möglich.

Verwendet man ein bereits in sich stabiles Vlies (1) ohne vorherige In-Line Vliesbildungseinrichtung, beispielsweise ein Spinnvlies oder ein Meltblown-Vlies, kann die Passage des Prägekalanders (6), der in Figur 3 gezeigt ist, entfallen.

Das erste, beheizte Kalanderwalzenpaar des Prägekalanders (6), bestehend aus einer Glatt- und einer Prägewalze (6a und 6b), verfestigt den Faserflor partiell mit der Gravur der Prägewalze (6b).

Wichtig ist dabei eine zunächst möglichst geringe Verfestigungsfläche, um im späteren Vliesstoff eine gute Ausprägung der gewünschten Struktur zu gewährleisten. Als günstig haben sich Verfestigungsflächen von 5% bis 15%, z.B. das Design NW 146 des Herstellers Casaretto, erwiesen.

Die für den Verfestigungsprozess notwendigen Parameter wie Druck und Temperatur sind in Abhängigkeit von der Produktionsgeschwindigkeit und der verwendeten Fasermischung zu wählen.

Im vorliegenden Beispiel lag der Druck, mit welchem die Kalanderwalzen aufeinander pressen bei 90 daN/cm und die Temperaturen bei 147 °C für die Prägewalze (6b) und 145 °C für die Glattwalze (6a).

Das so erhaltene Vlies (1), welches bereits eine gewisse Eigenfestigkeit aufweist, wird einem weiteren Kalander, dem Strukturkalander (7) zugeführt.

Der Strukturkalander (7) besteht nun wiederum aus einem beheizten Walzenpaar. Der Aufbau aus Glattwalze (7a) und Strukturwalze (7b) führt bei Behandlung des Vlieses (1) zum erfindungsgemäß strukturierten Vliesstoff (4), beispielsweise mit den Querschnittsformen A bis E aus Figur 2 beziehungsweise Figur 5.

Um die Querschnittsform B aus Figur 2 zu erreichen, kann auch eine Kombination aus zwei Strukturwalzen (7b) zum Einsatz kommen, wobei die Glattwalze (7a) durch eine Strukturwalze (7b) ersetzt wird.

Für das Beispiel wurde mit einem handelsüblichen Kalander mit Glattwalze (7a) und Strukturwalze (7b) gearbeitet.

Als Muster für die Strukturwalze (7b) wurde, gemäß der Figur 4, dabei ein umlaufendes Ringprofil mit Plateaus (9) mit einer Breite von 1,0mm verwendet. Diese Plateaus (9) weisen zueinander den Abstand (8) auf. Im vorliegenden Beispiel lag dieser bei 3,0mm. Die Tiefe (10) der Aussparungen zwischen Plateaus (9) lag bei 3,0mm. Der so erhaltene, erfindungsgemäß strukturierte Vliesstoff (4) zeigt bei seitlicher Betrachtung die Querschnittsform A aus Figur 2.

Die glatte Walze (7a) wurde auf eine Temperatur von 140°C und die Strukturwalze (7b) auf eine Temperatur von 115°C eingestellt. Der Anpressdruck betrug 80N/mm.

Das Vlies (1) wurde in dem vorgenannten Strukturkalander (7) behandelt, sodaß sich durch die Art der Gravur in den Bereichen der Stege (8) die erfindungsgemäßen Zonen geringerer Komprimierung (3), auch Mikropolster genannt, entstehen. An den Berührungsstellen der Plateaus (9) mit der Glattwalze (7a) entstehen die erfindungsgemäßen Zonen starker Komprimierung (2), auch Vertiefungen genannt.

Der aus der Behandlung des Vlieses (1) entstandene, erfindungsgemäße strukturierte Vliesstoff (4) weist nun eine Dicke von 0,62mm, gemessen nach ERT 30.5-99, Verfahren 5.2 mit Vorlast von 0,5 kPa, auf. Diese gemessene Dicke stellt auch gleichzeitig die Höhe der Mikropolster dar.

Im Vergleich zur Ausgangsdicke A des Vlieses (1) mit 0,94 mm entspricht dies einer Dickenreduzierung in Höhe von 34 % gegenüber der Ausgangsdicke A.

Die Dicke der Zonen starker Komprimierung (2) kann mit der oben genannten ERT-Methode nicht ermittelt werden. Da auch konventionelle Messuhren nicht über die erforderliche Feinheit an den Messfühlern verfügen, wurde die Materialdicke in diesen Zonen mikroskopisch, ohne Vorlast an Querschnitten des strukturierten Vliesstoffes (4) ermittelt. Diese lag für das Beispiel bei 0,22mm, was einer Reduzierung der Ausgangsdicke A in Höhe von 77% entspricht.

Die so erhaltenen, erfindungsgemäß strukturierten Vliesstoffe (4) können in einer Vielzahl von Anwendungen zum Einsatz kommen. Nachstehend sind nur einige genannt, wobei der Bereich der Aufnahme- und Verteilvliesstoffe näher betrachtet wird.

Der nach diesem Beispiel erhaltene, erfindungsgemäß strukturierte Vliesstoff (4) kann als Aufnahme- und Verteillage im Bereich der Hygieneprodukte wie Damen binden, Slipeinlagen oder Windeln eingesetzt werden.

Der Lagenaufbau von derartigen, körperflüssigkeiten-absorbierenden Produkten lässt sich wie folgt charakterisieren:
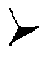 Dem Benutzer zugewandtes Topsheet aus z. B. thermobonded PP-Vliesstoff
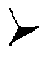 Aufnahme-/ Verteillage nach dem vorstehend beschriebenen Stand der Technik
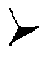 Saug-/ Speicherschicht aus beispielsweise Cellulose-Pulp mit SAP-Anteilen
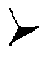 Backsheet aus z.B. PE-Folie

Die Funktion konventioneller, als Stand der Technik beschriebener Aufnahme-/ Verteillagen ist es nun, die Körperflüssigkeiten, nachdem sie durch das Topsheet penetriert sind, zunächst aufzunehmen und in sich möglichst großflächig zu verteilen, um dann die Körperflüssigkeiten innerhalb vertretbarer Zeit an die Saug-/ Speicherschicht zur Immobilisierung abzugeben.

Diese Funktion des Aufnehmes und Verteilens muss auch bei einer mechanischen Belastung und mehrmaliger Flüssigkeitsbeaufschlagung erhalten bleiben.

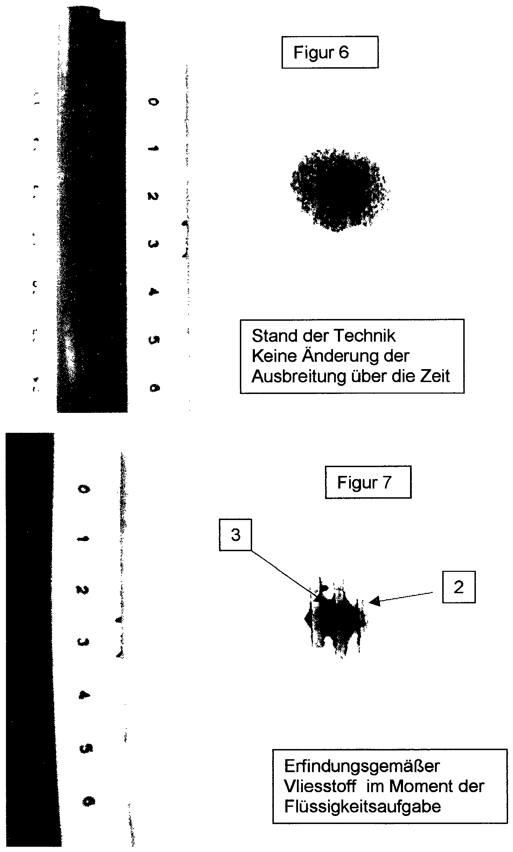

Betrachtet man Figur 6, so sieht man eine typische Verteilung von Körperflüssigkeiten bei Verwendung von Aufnahme-/ Verteillagen nach dem Stand der Technik.

Die Verteilung geschieht konzentrisch um den Aufgabepunkt herum, ist in der Aufnahme-/Verteillage nur ungenügend und geschieht hauptsächlich in der eigentlich zu entlastenden Saug-/ Speicherschicht.

Die Figuren 7 bis 9 zeigen eine vergleichsweise durchgeführte Prüfung mit einem als Aufnahme-/ Verteillage eingesetzten erfindungsgemäßen strukturierten Vliesstoff.

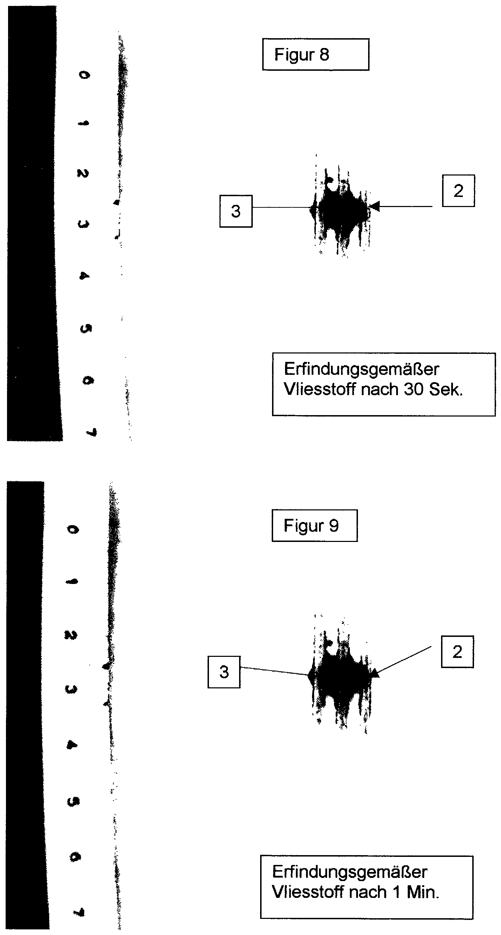

Deutlich erkennbar ist die Aufnahme und Zwischenspeicherung der Körperflüssigkeiten in den Zonen geringer Komprimierung (3) und in den Figuren 8 und 9 die kontrollierte Verteilung entlang der Zonen starker Komprimierung (2).

Dabei kann über die Auswahl der eingesetzten Fasermaterialien, ob nun hydrophil oder semi-hydropob, speziell aber durch den Titer der Fasern, eine Anpassung des Aufnahme-/ Verteilverhaltens des erfindungsgemäß strukturierten Vliesstoffes (4) an die zu erwartenden Mengen an Körperflüssigkeiten geschehen.

Für große Mengen von Körperflüssigkeiten, beispielsweise in der Erwachseneninkontinenz, werden gröbere Fasern bevorzugt eingesetzt, dies bedeutet einen Titerbereich von 7 bis 12 dtex. Bei Babywindeln ist der Titerbereich 3,3 bis 6,7 dtex sinnvoll.

Wie eingangs erwähnt, ist nicht nur die einmalige Aufnahme-/ und Verteilwirkung entscheidend, sondern auch die Resistenz gegenüber mechanischer Belastung, speziell der Druckbelastung.

Hier wird ein Rückformvermögen der zum Einsatz kommenden Aufnahme-/ Verteillage gefordert, die bei dem erfindungsgemäßen, strukturierten Vliesstoff (4) durch die Mikropolster, gewährleistet wird.

Das heißt, dass bei Wegfall der Druckbelastung die flachgedrückten Faserlagen der Zonen geringer Komprimierung (3) innerhalb des erfindungsgemäßen Vliesstoffes (4) sofort eine Rückstellkraft aufbauen und ihre ursprüngliche Form, die sogenannten Mikropolster wieder einnehmen.

Die Rückstellkraft wird durch die Wahl der Gravur, speziell durch die Abstände (8) der Plateaus (9) zueinander und durch die Kräuselung der eingesetzten Faserstoffe für das Vlies (1) beeinflusst.

Es zeigte sich, dass der Abstand (8) am besten als ganzzahliges Vielfaches der Länge eines Kräuselbogens der Faser mit dem grössten Titer gewählt wird.

Im oben beschriebenen Beispiel war der Abstand (8) das Dreifache einer Kräuselbogenlänge. Zu bedenken ist jedoch, dass, je größer der Abstand (8) gewählt wird, desto geringer die erzielbare Rückstellkraft wird.

Durch die Ausnutzung der Rückstellkraft der Mikropolster und die in den Zonen starker Komprimierung (3) vorhandene Kapillarwirkung, bleibt selbst nach mehrmaliger Flüssigkeitsbeaufschlagung die Aufnahme- und Verteilwirkung erhalten. Dies sogar auch trotz der Tatsache, dass die Avivage der eingesetzten Faserstoffe großteils abgewaschen wird.

Wie sich erfindungsgemäße, strukturierte Vliesstoffe (4) im vorstehend beschriebenen Verhalten vom Stand der Technik abheben, kann der nachstehenden Tabelle 1 entnommen werden.

Die eingesetzten Vliese (1), welche alle für sich als Aufnahme-/und Verteilvlies geeignet sind wurden ohne und mit eingebrachter Struktur getestet. Als Struktur wurde die im Beispiel beschriebene, das heißt Rillen mit einer Plateaubreite von 1mm und einem Abstand von 3mm zueinander verwendet.

Der Strike Through und auch der Rewet wurden gemäß der gängigen ERT Methoden 150 und 151 ermittelt, wobei der folgende Aufbau verwendet wurde:
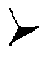 Elektrode bzw. Gewicht
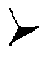 Topsheet aus 100% PP-Faser, 2,2 dtex, hydrophil
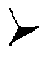 Aufnahme-/Verteillage aus Stand der Technik und erfindungsgemäß
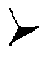 Saugpapier H&V, Typ ERT FF3 Strike Through/Wetback, smooth side up

**Tabelle 1: Vergleich von Vliesstoffen als Auf-/Verteillagen nach dem Stand der Technik und erfindungsgemäßer Ausführung**

| | | Flächengewicht | Dicke | 1. Strike Through | 3. Strike Through | 5. Strike Through | 1. Rewet | 3. Rewet | 5. Rewet |
|---|---|---|---|---|---|---|---|---|---|
| | Mischung | g/m² | 0.5 kPa mm | s | s | s | g | g | g |
| Vlies A | 100% Bico PET/CoPET 5,3 dtex | 38,1 | 1,23 | 1,2 | 1,6 | 2,0 | 0,12 | 0,26 | 0,5 |
| **Vlies A +Struktur** | **100% Bico PET/CoPET 5,3 dtex** | **56,5** | **0,74** | **1,4** | **1,6** | **1,6** | **0,08** | **0,07** | **0,08** |
| Vlies B | 100% Bico PP/PE 3,7 dtex | 42,6 | 2,17 | 1,0 | 1,2 | 79,5 | 0,12 | 0,18 | 1,25 |
| **Vlies B +Struktur** | **100% Bico PP/PE 3,7 dtex** | **46,0** | **1,2** | **1,5** | **1,9** | **2,8** | **0,11** | **0,10** | **0,11** |
| Vlies C | **30%Bico PET 5,3 dtex** / **70% PET 7.0dtex** | 64,3 | 0,94 | 1,8 | 2,2 | 3 | 0,10 | **0,11** | **0,11** |
| **Vlies C +Struktur** | **30% Bico PET 5,3 dtex / 70% PET 7.0dtex** | **64,4** | **0,62** | **1,5** | **2,1** | **2,2** | **0,09** | **0,09** | **0,09** |
| Nach Stand der Technik EP 0810078 | 30%Bico PET 5,3 dtex / 70% PET 7.0dtex | 64,8 | 0,66 | 1,9 | 3,8 | 4,1 | 0,10 | 0,4 | 1,99 |

Die Prüfungen wurden fünfmal im Abstand von 15 Minuten wiederholt, wobei zwischen den Prüfungen der Aufbau mit einem Gewicht von 4 kg belastet wurde. Nach jeder Rewet-Ermittlung wurde das Saugpapier gewechselt.

Bei den Vliesstoffen ohne Struktur zeigte sich, dass bei mehrmaliger Belastung die Verteilwirkung abnimmt, die Flüssigkeit nicht zwischengespeichert wird und dann durch das Topsheet zurückpenetriert. Dies zeigt sich durch erhöhte Rewet-Werte.

Das erfindungsgemäße Vlies mit Struktur zeigt aufgrund der Mikropolster genug Resistenz gegen die Druckbelastung und verliert daher die Aufnahme- und Verteilwirkung nicht, was sich in geringer Strike-Through- Zeiten und Rewet-Werten bemerkbar macht. Anders ist dies beim Stand der Technik, speziell letztes Beispiel der Tabelle 1. Das eingesetzte Vlies mit trapezförmigen Querschnitt zeigt keinerlei Resistenz gegen Druckbelastung und fällt bereits bei der Ermittlung der Dicke zusammen.

Die Dickenreduzierung beim Einbringen der erfindungsgemäßen Struktur macht sich auch positiv in der Höhe des Gesamtaufbaus einer Windel bemerkbar. Auch ist die verbesserte Resistenz gegenüber von Druckbelastungen durch die Mikropolster positiv bei der Komprimierung von beispielsweise mit dieser Aufnahme- und Verteillage ausgestatteten Windeln.

Nach einer Pressverpackung in die Verkaufsverpackungen der Windeln, zeigen Windeln mit dem erfindungsgemäßen Vliesstoff (4) bessere Ansaugzeiten als Windeln mit Materialien des Standes der Technik.

Überraschenderweise zeigte sich, das diese Art strukturierter Vliesstoffe auch noch in anderen Bereichen vorteilhaft angewendet werden können.

So kann sich bei Anwendung des erfindungsgemäßen Vliesstoffes als Reinigungstuch in den Vertiefungen zwischen den Mikropolstern Schmutz einlagern, der dann in die fasrigen Mikropolster migriert und gespeichert wird. Vorteilhaft ist diese Eigenschaft speziell bei beim Reinigen öliger, glatter Oberflächen.

Die Einzelfasern sind bei der Verwendung kardierter Vliese (1) durch die strukturgebende Prägung des Kalanders (7) gut eingebunden und können nicht aus dem Verbund ausgelöst werden, das Reinigungstuch ist quasi "fusselfrei".

Im Bereich der Filtration von Fluiden haben sich erfindungsgemäße Vliesstoffe hauptsächlich durch die gerichtete und durch die Wahl der Komprimierungsstruktur steuerbare Verteilung der Fluide und die gezielte Abscheidung der darin enthaltenen Verunreinigungen ausgezeichnet.

Hier kann zum einen ein staubbeladenes Gas gezielt filtriert werden, zum anderen ist dies aber auch in der Flüssigkeitsfiltration machbar.

Die Verwendung von auf der Meltblowntechnologie basierenden Vliesen (1) hat sich hier bewährt, es sind aber auch Spinnvliese anwendbar.

Ebenso können derartig strukturierte Vliesstoffe (4) im Baubereich, speziell bei Abdichtungsbahnen für Dächer eingesetzt werden, da hier eine gerichtete Ableitung überschüssigen Wassers aus dem Überlappungsbereich der Abdichtungsbahnen nach dem Verlegen gewünscht ist. Andererseits ist bei wasserdampfdurchlässigen Unterspannbahnen eine möglichst große Spreitung der zu verdampfenden Flüssigkeitsmenge gefordert. Dies wird durch eine zwischengeschaltete Lage des erfindungsgemäß strukturierten Vliesstoffes (4) gewährleistet.

Auch in der Bekleidungsherstellung ist die Verwendung derartiger Funktionsvliese sinnvoll. Hier ist die Aufgabenstellung der schnelle Abtransport von Körperflüssigkeiten, hier Schweiß, weg von der Hautoberfläche und vom Entstehungsort, zum Beispiel der Achselpartie, durch die Wattierung hin zur Oberfläche des Bekleidungsteils und trotzdem ein beständiges Vlies gegen Scheuerbeanspruchen zu haben.

Insbesondere bei zonal auftretender Schweiß-Entwicklung fördert die Verteilfunktion des erfindungsgemäß strukturierter Vliesstoffes (4) den Abtransport des Schweißes durch die folgenden Lagen des Bekleidungsteiles, insbesondere durch eventuelle semipermeable Membranen.

Hier macht sich die Spreitwirkung des erfindungsgemäßen Vliesstoffes (4) vorteilhaft bemerkbar, da der Schweiß über eine größere Fläche verteilt wird und somit schneller durch die Membran migrieren kann, der Träger dadurch schneller ein trockenes Hautgefühl hat.

Die Anwendung der erfindungsgemäß strukturierten Vliesstoffe (4) ist also in allen Bereich sinnvoll, die eine gesteuerte, zielgerichtete Abführung von Fluiden, gleich welcher Art, bei gleichzeitiger Resistenz gegen mechanische Belastung verlangen.

## Patentansprüche

1. Aufnahme- und Verteilvliesstoff für Körperflüssigkeiten, wobei der Vliesstoff thermisch kalanderverfestigt ist und eine Struktur aus in den verfestigten Vliesstoff eingebrachten Vertiefungen aufweist,
**dadurch gekennzeichnet, dass**
der Vliesstoff (1) der Ausgangsdicke A zonal unterschiedlich stark, dauerhaft thermisch komprimiert ist und somit Zonen starker Komprimierung (2) enthält, deren Dicke höchstens 30% der Ausgangsdicke A beträgt und Zonen geringerer Komprimierung (3) als Mikropolster enthält, deren Dicke mindestens 5% gegenüber der Ausgangsdicke A verringert ist,
der Vliesstoff (1) Zonen starker Komprimierung (2) in Form parallel zueinander verlaufender, durchgehender Vertiefungen enthält,
die Zonen stärkerer Komprimierung (2) und die Zonen schwächerer Komprimierung (3) in einem Dickenverhältnis von wenigstens 1:2 und höchstens von 1:50 zueinander stehen und
dass die Zonen stärkerer Komprimierung (2) und die Zonen schwächerer Komprimierung (3) in einem Breitenverhältnis von wenigstens 5:1 und höchstens 1:50 zueinander stehen.

## Claims

1. Nonwoven absorbing and dispersing fabric for body fluids, the nonwoven fabric being thermally calender-fixed and having a structure of indentations introduced into the consolidated nonwoven fabric,
**characterized in that**
the nonwoven fabric (1), of initial thickness A, is greatly and permanently thermally compressed in differing degrees by zones, and thus contains zones of great compression (2), whose thickness is no more than 30% of the initial thickness A, and contains zones of lesser compression (3) as micro-bolsters, whose thickness is reduced by at least 5% compared to the initial thickness,
the nonwoven fabric (1) contains zones of great compression (2) in the form of continuous indentations running parallel to one another,
the zones of greater compression (2) and the zones of lesser compression (3) have a thickness ratio of at least 1:2 and no more than 1:50, and
the zones of greater compression (2) and the zones of lesser compression (3) have a width ratio of at least 5:1 and no more than 50:1.

## Revendications

1. Non-tissé d'absorption et de dispersion pour des liquides corporels, où le non-tissé est renforcé par un calandrage thermique et présente une structure à base d'alvéoles rapportées dans le non-tissé renforcé,
**caractérisé en ce que**
le non-tissé(1) d'épaisseur de départ A est comprimé thermiquement de manière durable avec une force différente selon les zones, et contient ainsi des zones de forte compression (2), dont l'épaisseur se situe au maximum à 30 % de l'épaisseur de départ A, et des zones de moindre compression (3), sous la forme d'un micro matelassage, dont l'épaisseur est au moins diminuée de 5 % par rapport à l'épaisseur de départ A,
le non-tissé (1) contient des zones de forte compression (2) sous la forme de creux s'étendant tout le long, parallèles les uns aux autres,
les zones de forte compression (2) et les zones de moindre compression (3) ont un rapport en épaisseurs d'au moins 1 : 2 et d'au plus 1 : 50 les unes par rapport aux autres, et
**que** les zones de forte compression (2) et les zones de moindre compression (3) ont un rapport en largeurs d'au moins 5 : 1 et d'au plus 1 : 50 les unes par rapport aux autres.
